# EUROPEAN PATENT SPECIFICATION

(11) **EP 1 278 515 B1**
(45) Date of publication and mention of the grant of the patent: **03.01.2007**
(21) Application number: 01926063.7
(22) Date of filing: 27.04.2001
(51) Int. Cl.: A61K 9/50, A61K 31/522, A61P 31/22

(54) **PHARMACEUTICAL FORMULATION COMPRISING CARRIER BEADS COATED WITH A LAYER OF VALACICLOVIR**
PHARMAZEUTISCHE FORMULIERUNG ENTHALTEND TRÄGERKÜGELCHEN MIT AUFGEBRACHTER SCHICHT AUS VALACICLOVIR
PREPARATION PHARMACEUTIQUE COMPRENANT DES MICRO-BILLES DE SUPPORT RECOUVERTES D'UNE COUCHE DE VALACICLOVIR

(30) Priority: 28.04.2000 GB 0010446
(43) Date of publication of application: 29.01.2003
(73) Proprietor: Glaxosmithkline Kabushiki Kaisha, Tokyo 151-8566 (JP)
(72) Inventor: KAWAMURA, Koho, Imaichi-shi, Tochigi 321-1274 (JP); MISHIMA, Yasuhiro, Imaichi-shi, Tochigi 321-1274 (JP); OHTSUKA, Kiyoshi, Imaichi-shi, Tochigi 321-1274 (JP); SUGIBAYASHI, Nobuya, Imaichi-shi, Tochigi 321-1274 (JP); SUGISAWA, Keiichi, Imaichi-shi, Tochigi 321-1274 (JP); YANAGIHARA, Hitomi, Imaichi-shi, Tochigi 321-1274 (JP); YASUDA, Makio, Imaichi-shi, Tochigi 321-1274 (JP)
(74) Representative: Breen, Anthony Paul
(86) International application number: PCT/JP2001/003738
(87) International publication number: WO 2001/082905

(56) References cited:
- EP-A- 0 381 174
- WO-A-01/37808
- US-A- 4 874 613

## Description

The present invention relates to a pharmaceutical formulation of the antiviral drug valaciclovir, or a pharmaceutically acceptable derivative thereof, useful in the treatment of disease in mammals, including humans. The invention also relates to a process for the preparation of such a formulation.

The compound 9-[(2-hydroxyethoxy)methyl]guanine, otherwise known as acyclovir possesses potent antiviral activity and is widely used in the treatment and prophylaxis of viral infections in humans, particularly infections caused by the herpes group of viruses (see, for example, Schaeffer *et al.* Nature, 272, 583-585 (1978), UK Patent No. 1523856, US Patent No. 4,199,574). However, acyclovir is poorly absorbed from the gastrointestinal tract upon oral administration and this low bioavailability means that multiple high doses of oral drug may need to be administered, especially for the treatment of less sensitive viruses or infections in order to achieve and maintain effective anti-viral levels in the plasma.

The L-valine ester of acyclovir [2-(2-amino-1,6-dihydro-6-oxo-purin-9-yl)methoxy]ethyl L-valinate (hereinafter referred to as valaciclovir) has been shown to possess much improved bioavailability whilst retaining the anti-viral properties of acyclovir. A prefered form of this compound is its hydrochloride salt which is herein referred to as valaciclovir hydrochloride. Valaciclovir and its salts including the hydrochloride salt are disclosed in US Patent No. 4,957,924, European Patent No. 0308065 and Beauchamp et al, Antiviral Chemistry and Chemotherapy, 3(3), 154-164 (1992).

Valaciclovir and methods for its preparation are disclosed in inter alia, European Patent No. 0308065 and PCT publication WO 96/22082, both incorporated herein by reference. European Patent No. 0308065 generically discloses formulations for oral administration including granules, however no formulations for oral granules are specifically disclosed therein WO 96/22082, discloses the formulation of valaciclovir into tablets. A further PCT publication, WO 96/32097, discloses sustained release compositions for non-amorphous active ingredients such as valaciclovir.

US 4,874,613 (Hsiao) discloses a pharmaceutical dosage unit comprising a plurality of subdosage units being pellets disposed within a container. Each pellet has an average diameter not greater than about 1 mm and includes (a) an inner core particle coated with an orally active pharmaceutical ingredient, (b) a first layer surrounding the core consisting of an inert clay or water-soluble polymer, and (c) a second layer surrounding the first layer comprising a cationic copolymeric acrylate resin and a basic compound being calcium carbonate, aluminium hydroxide and magnesium carbonate. In US 4,874,613, the first or inner layer of a biologically inert filler is stated to serve the primary purpose of masking the taste of the active ingredient while not interfering with its release.

WO 01/37808 A1 (Lipocine, Inc.) discloses a pharmaceutical composition comprising a solid carrier, the solid carrier comprising an encapsulation coat on a substrate, the encapsulation coat comprising at least one pharmaceutical active ingredient (e.g. valaciclovir) and at least one hydrophilic surfactant.

Valaciclovir has proven difficult to formulate. Certain difficulties of formulating valaciclovir are described in WO 96/22082. For example, WO 96/22082 explains that it is difficult to obtain a tablet of sufficient hardness and friability for pharmaceutical handling and for film coating. One problem is that valaciclovir has "adhesive" properties in that it can stick to the tablet dies and therefore needs to be efficiently lubricated. The formulation of granules of valaciclovir was also found to be problematic due to the adhesive properties of valaciclovir and also to problems relating to the pH dependent dissolution rate and discolouration of the granules. Granules of valaciclovir itself tend to be fragile and have a low coating efficacy. Furthermore, there is a need to mask the bitter taste of valaciclovir. It is therefore an object of the invention to provide a suitable granule formulation for valaciclovir.

We have found that pharmaceutical formulations of the present invention are particularly suitable for formulating oral granules comprising valaciclovir, or a pharmaceutically acceptable derivative thereof. Oral granules have certain advantages also associated with tablets, i.e. they can easily be stored and are stable over long periods, but oral granules are preferred in certain countries as they represent advantages over tablets in that the dose can be more easily manipulated depending on the body weight of the patient. They may also be more easily administered for those patients who have difficulty swallowing tablets, e.g. granules are convenient for oral administration to children and the elderly.

The present invention accordingly provides a pharmaceutical formulation, comprising carrier beads coated with one or more layers of pharmaceutically acceptable substances, wherein, at least one layer is a drug coat layer comprising valaciclovir, or a pharmaceutically acceptable derivative thereof, as the active ingredient, and wherein the pharmaceutical formulation further comprises a mask coat layer.

As used herein "pharmaceutically acceptable derivative" means any pharmaceutically acceptable salt of valaciclovir which, upon administration to the recipient, is capable of providing (directly or indirectly) valaciclovir or an antivirally active metabolite or residue thereof.

Pharmaceutically acceptable salts of valaciclovir are preferably acid addition salts derived from an appropriate acid, e.g. hydrochloric, sulphuric, phosphoric, maleic, fumaric, citric, tartaric, lactic, acetic or p-toluenesulphonic acid. A particularly preferred salt is the hydrochloride salt of valaciclovir.

Valaciclovir or pharmaceutically acceptable derivatives thereof are hereinafter referred to generally as the 'active ingredient'.

Suitable formulations contain 1 to 90% by weight of active ingredient. Preferred formulations contain from 25 to 75% by weight of active ingredient, desirably from 50 to 60%.

In the present invention the active ingredient is provided on carrier beads. The use of a solid core (carrier beads) makes it possible to achieve excellent coating efficacy due to the uniform shape and size of the solid core. A carrier bead comprises a pharmaceutically acceptable substance other than the active ingredient which provides a surface onto which one or more layers of pharmaceutically acceptable substances, at least one of which comprises the active ingredient, may be coated. Suitable carrier beads may comprise cellulose (e.g. Celluphia CP305 or Celluphia CP203) such as microcrystalline cellulose, non-pareils. (carrier beads made of sucrose) (e.g. Freund Industry), lactose, or starch. Preferably the carrier beads comprise cellulose (e.g. Celluphia CP305, Asahi Chemical Industry). Celluphia CP305 is pure cellulose. Preferably the carrier beads are present in amounts of 1 to 50% by weight of the total formulation, more preferably 20 to 40%. Preferably the range of particle sizes of the solid core is 300 to 500µm, more preferably the average particle size of the solid core is 400µm in diameter.

In a preferred aspect of the invention, the drug coat layer further comprises a component which acts as a binder. Suitable binders include cellulosics (e.g. Hydroxy Propyl Cellulose (HPC-L, Nippon Soda), Hydroxy Propyl Methyl Cellulose (HPMC)), starch and polyvinylpyrrolidone (e.g. PVP K30, PVP K90). A particularly preferred binder, providing strong binding effects with valaciclovir and good coating efficiency, is polyvinylpyrrolidone (PVP). PVP may be PVP K30 (a linear polymer of 1-vinyl-2-pyrrolidone having an average molecular weight of about 40000) or PVP K90 (a linear polymer of 1-vinyl-2-pyrrolidone having an average molecular weight of about 1200000) (e.g. ISP, BASF). Particularly preferred is polyvinylpyrrolidone grade K30. Preferably the binder is present in amounts of 1 to 30% by weight of the total formulation, more preferably 1 to 10%.

As valaciclovir has a particularly unpleasant taste, the formulation of the present invention further comprises a mask coat layer. Preferably the mask coat layer comprises a polymer, a lubricant and a binder/dissolution enhancer. Suitable polymers include Eudragit (Methacrylic acid copolymer as described in Japanese Pharmaceutical Excipients, 1998 (e.g. Eudragit NE30D, L30D55, L10055, L100, E100, S100, RS100L, RS30DL, RS100, or RS30D)); Cellulosics(HPC, HPMC, Hydroxy Propyl Methyl Cellulose Phthalate (HPMCP), Ethyl Cellulose (EC), Carboxy Methyl Ethyl Cellulose (CMEC), Cellulose Acetate Phthalate (CAP), Hydroxy Ethyl Cellulose (HEC)); wax (glyceryl monostearate etc.); gelatin and shellac. A preferred polymer is a methacrylic acid copolymer e.g. Eudragit, most preferably Eudragit NE30D. Eudragit NE30D offers the advantage of pH-independent solubility. Preferably the polymer is present in amounts 1 to 30% by weight of the total formulation, more preferably 1 to 10%. Suitable lubricants include talc, kaolin, magnesium stearate and stearic acid. A preferred lubricant is talc. Preferably the lubricant is present in amounts 1 to 30% by weight of the total formulation, more preferably 1 to 10%. Suitable binder/dissolution enhancers include HPC-L, PVP, starch, HPMC, Polyethylene glycol 6000 (PEG 6000), Polysorbate 80, Triacetin, Titanium Dioxide and Sodium Lauryl Sulphate. Preferably the binder/dissolution enhancer is HPC-L. Preferably the binder/dissolution enhancer is present in amounts 0.1 to 20% by weight of the total formulation, more preferably 0.1 to 5%.

Preferably a terminal blend is applied to the coated carrier beads to make final granules. The terminal blend helps to prevent the agglomeration and adhesion of the final granules which is associated with the adhesive property of valaciclovir. The terminal blend comprises one or more lubricants. Suitable lubricants are talc, aerosil, kaolin, magnesium stearate and stearic acid. Preferred lubricants are talc and aerosil. Talc is a purified, hydrated, magnesium silicate, approximating to the formula Mg₆(Si₂O₅)₄(OH)₄. A suitable talc is CROWN TALC KYOKU PP (Matsumura Industrial). Preferably the terminal blend comprises 0.1 to 20% by weight of the total formulation, more preferably 0.1 to 10%.

The formulations of the invention may, if desired, further include one or more pharmaceutically acceptable carriers or excipients. All such excipients must be "pharmaceutically acceptable" in the sense of being compatible with the other ingredients of the formulation and not injurious to the patient. Pharmaceutically acceptable excipients may include colours, flavours e.g. menthol, sweeteners e.g. mannitol, and other excipients known to those skilled in the art.

A preferred formulation of the invention comprises:
(a) carrier beads, comprising from 1 to 50% by weight of the total formulation;
(b) a drug coat layer comprising from 1 to 90% by weight of the total formulation comprising valaciclovir, or a pharmaceutically acceptable derivative thereof, and from 1 to 30% by weight of the total formulation of a component which acts as a binder;
(c) a mask coat layer comprising from 1 to 30% by weight of the total formulation of a polymer, from 1 to 30% by weight of the total formulation of a component which acts as a lubricant, and from 0.1 to 20% by weight of the total formulation of a component which acts as a binder/dissolution enhancer; and
(d) a terminal blend comprising from 0.1 to 20% by weight of the total formulation of a component which acts as a lubricant.

An especially preferred formulation of the invention comprises:
(a) carrier beads, comprising from 20 to 40% by weight of the total formulation;
(b) a drug coat layer comprising from 25 to 75% by weight of the total formulation comprising valaciclovir, or a pharmaceutically acceptable derivative thereof, and from 1 to 10% by weight of the total formulation of a component which acts as a binder;
(c) a mask coat layer comprising from 1 to 10% by weight of the total formulation of a polymer, from 1 to 10% by weight of the total formulation of a component which acts as a lubricant and from 0.1 to 5% by weight of the total formulation of a component which acts as a binder/dissolution enhancer, and
(d) a terminal blend comprising from 0.1 to 10% by weight of the total formulation of a component which acts as a lubricant.

A preferred specific formulation of the invention comprises:
(a) cellulose carrier beads, comprising about 30.6% by weight of the total formulation;
(b) a drug coat layer comprising about 55.6% by weight of the total formulation of valaciclovir hydrochloride, and about 4.2% by weight of the total formulation of polyvinylpyrrolidone as a binder;
(c) a mask coat layer comprising about 4.11% by weight of the total formulation of methacrylic acid copolymer, about 4.11 % by weight of the total formulation of talc as a lubricant, and about 0.89% by weight of the total formulation of hydroxypropylcellulose as a binder/dissolution enhancer; and
(d) a terminal blend comprising about 0.25% by weight of the total formulation of talc and 0.25% by weight of the total formulation of aerosil which act as lubricants.

It is to be understood that the present invention covers all combinations of particular and preferred groups described hereinabove.

To meet the standard on granules defined in the Japanese Pharmacopoeia, it is preferable that:
(i) no granules have a particle size greater than 1700µm.
(ii) no more than 5% by weight of the granules have a particle size greater than 1400µm. Preferably no more than 4% by weight of the granules have a particle size greater than 1400µm, for example, no more than 3% by weight. Desirably, less than 0.5% of the granules have a particle size greater than 1400µm.
(iii) no more than 15% by weight of the granules have a particle size smaller than 355µm. Preferably no more than 10% by weight of the granules have a particle size smaller than 355µm, for example, no more than 5% by weight. Desirably, less than 0.5% of the granules have a particle size smaller than 355µm. Preferably up to 98 to 100% by weight of the granules of the invention have such a particle size.

Particle size is determined by the Particle Size Distribution Test for Granules, The Japanese Pharmacopoeia, Thirteenth Edition 1996.

Formulations of the present invention preferably have the following properties:
(i) no granules have a particle size greater than 1700µm.
(ii) at least 95% by weight of the granules have a particle size of from 355µm to 1400µm.
(iii) no more than 0.5% by weight of the granules have a particle size smaller than 355µm.
   Preferably the range of particle sizes for the finished granules is 355 to 850µm, more preferably the average particle size of the finished granules is 800µm.

The present invention provides a pharmaceutical formulation for use in medical therapy, e.g. in the treatment of a viral disease in an animal, e.g. a mammal such as a human. The compound is especially useful for the treatment of diseases caused by various DNA viruses, such as herpes infections, for example, herpes simplex 1 and 2, varicella zoster, cytomegalovirus, Epstein-Barr viruses or human herpes virus-6 (HHV-6) as well as diseases caused by hepatitis B. The active compound can also be used for the treatment of papilloma or wart virus infections, and may furthermore be administered in combination with other therapeutic agents, for example, with zidovudine, to treat retroviral associated infections in particular HIV infections.

In addition to its use in human medical therapy, the active compound can be administered to other animals for treatment of viral diseases, e.g. to other mammals. In particular, the compound is active against herpes simplex virus which causes herpetic keratitis in rabbits, herpetic encephalitis in mice, and cutaneous herpes in guinea pigs.

For each of the above-indicated utilities and indications the amount required of the active ingredient will depend on a number of factors including the severity of the condition to be treated and the identity of the recipient and will ultimately be at the discretion of the attendant physician or veterinarian. In general, however, for each of these utilities and indications, a suitable effective dose will be in the range 1 to 150mg per kilogram bodyweight of recipient per day, preferably in the range 5 to 120mg per kilogram bodyweight per day (Unless otherwise indicated, all weights of the active ingredient are calculated with respect to the free base valaciclovir). The desired dose is preferably presented as one, two, three or four or more sub-doses administered at appropriate intervals throught the day. These sub-doses may be administered in unit dosage forms, for example, containing about 50 to 2000mg, preferably about 250, 500, 1000 or 2000mg of active ingredient per unit dose form, most preferably about 500mg of active ingredient per unit dose form.

The following dosage regimes are given for guidance: treatment of herpes simplex virus types 1 and 2 infection: total daily dose of about 1 or 2g administered at 500mg twice a day or 1g twice a day for 5 to 10 days; suppression of herpes simplex virus types 1 and 2 infections: total daily dose about 250mg to 1g for about one to ten years (depending on the patient, age or length of treatment); treatment of varicella zoster virus infections (for example shingles): daily dose about 3g administered at 1g three times a day for seven days; supression of cytomegalovirus infections: total daily dose about 8g administered at 2g four times a day. For transplant patients this daily dose is administered for three to six months for the period at risk; and for HIV positive patients said daily dose is administered as usually indicated for improving quality of life, for example, for two years or more.

Early results now indicate that valaciclovir can be used in the effective suppression of recurrent genital herpes at a once daily dose of from about 200mg to about 1000mg for an effective treatment period. The most likely daily dosages are 250mg, 500mg or 1000mg. For example, a valaciclovir dose of 500mg may be provided in 1g of granules.

The term "treatment" and derivatives such as "treating" as used herein includes both treatment and prophylaxis.

A further aspect of the invention provides a process for preparing granules comprising coating a carrier bead with a drug coat layer comprising valaciclovir, or a pharmaceutically acceptable derivative thereof, as the active ingredient, and further coating with a mask coat layer and where appropriate with a terminal blend.

The invention also provides a process for preparing a pharmaceutical formulation as defined herein comprising coating a carrier bead with a drug coat layer comprising valaciclovir, or a pharmaceutically acceptable derivative thereof, and further coating with a mask coat layer. In the process for preparing the pharmaceutical formulation, the drug coat layer is preferably applied to the carrier bead using an aqueous solvent.

Methods for making valaciclovir are disclosed in European Patent No. 0308065. Valaciclovir hydrochloride (anhydrous crystalline form) can be made as described in WO 96/22082, the contents of which are incorporated herein by reference.

Carrier beads may be granulated into beads using an aqueous solution or organic solvents such as ethanol or isopropyl alcohol (IPA). Preferably the carrier beads are granulated using an aqueous solution, more preferably the aqueous solution is water. Celluphia round beads are granules of microcrystalline cellulose powder, granulated with water (Asahi Chemical Industry). Granulation is carried out using a suitable granulator, as known to a person skilled in the art. The resulting granules may be passed through a sieve to select particle size. Carrier beads of an average particle size 300 to 500µm are preferred, more preferably the average particle size is 400µm in diameter.

Layers of pharmaceutically acceptable substances may be applied to carrier beads using a non-aqueous system with ethanol or IPA as a solvent, or alternatively using an aqueous system, e.g. purified water. The aqueous system offers the advantage that the process may be safer and cheaper. A preferred feature of the invention is therefore the use of water as a solvent to form a layer of the active ingredient. The water used is preferably purified water, purified that is according to the standards of the Japanese Pharmacopoeia.

A drug coat layer may be prepared by first heating a solvent, e.g. purified water, suitably to 65°C, and dissolving the active ingredient and binder in the solvent. The solution is then cooled to room temperature and filtered. The obtained aqueous suspension (the active ingredient and binder) is sprayed onto carrier beads and the coated carrier beads are dried in a rotated fluid bed granulator.

A mask coat layer may be prepared by mixing the polymer, lubricant and binder/dissolution enhancer and a solvent, e.g. purified water, to form a suspension and filtering the suspension. The mask coat suspension is sprayed onto drug coated beads and the coated carrier beads are dried in a rotated fluid bed granulator.

The dried granules are subjected to sieving to ensure that the appropriate particle size requirements are met. Preferably the range of particle sizes for the finished granules is 355 to 850µm. More preferably, the average particle size of the finished granules is 800µm.

Finally, the granules are blended with lubricants in a suitable blender as known to a person skilled in the art.

The resulting granules may be taken directly or incorporated into pharmaceutical compositions for oral administration. The granules may be administered in an inert liquid vehicle so as to form a drench, or in a suspension with a water or oil base. For example, the granules may be administered in a syrup. Preferably, the granules are taken directly.

The formulation may be introduced into a container which is then closed. The container may be sealed. It may be a single-dose or multi-dose container. The container may be bottles, jars, bags or sachets. Sachets, especially foil sachets (foil-foil blisters), are particularly suitable for single dose packaging. Bottles, particularly high density polyethylene (HDPE) bottles, are particularly suitable for multi-dose packaging.

The following examples illustrate aspects of this invention but should not be construed as limiting the scope of the invention in any way.

### Example 1

| Process | Ingredients | | Amount (%) |
|---|---|---|---|
| Drug Coat | Carrier beads: Cellulose (Celluphia CP305) | | 30.6 |
| | Valaciclovir HCI | | 55.6 |
| | (as free base) | | (50.0) |
| | Binder: polyvinylpyrrolidone (PVP K30) | | 4.2 |
| Mask Coat | Polymer: Methacrylic acid copolymer | | 4.11 |
| | (Eudragit NE30D) | | (as solid) |
| | Lubricant: Talc | | 4.11 |
| | Binder/dissolution enhancer: Hydroxypropyl cellulose (HPC-L) | | 0.89 |
| Terminal | Lubricants: | Talc | 0.25 |
| Blend | | Aerosil | 0.25 |
| Total | | | 100 |

### 1) Drug Coating

Purified water was heated to 65 °C and PVP K30 and valaciclovir HCI were added to the water and dissolved in a stainless mixing tank with jacket heater. Then, the solution was cooled to room temperature. The obtained aqueous suspension was filtered and sprayed onto Celluphia CP305, and dried in a rotated fluid bed granulator.

### 2) Mask Coating

HPC-L was dissolved in purified water and Talc and Eudragit NE30D were added to the solution, and dispersed in a stainless mixing tank. The coating suspension was filtered and sprayed onto drug coated beads, and dried in the same machine.

### 3) Terminal Blend

The mask coated beads, Talc and Aerosil were blended in a V-shaped blender.

### 4) Packaging

The obtained final granules (beads) were filled in bottles, jars, bags or sachets. Appearance: White Round Granules

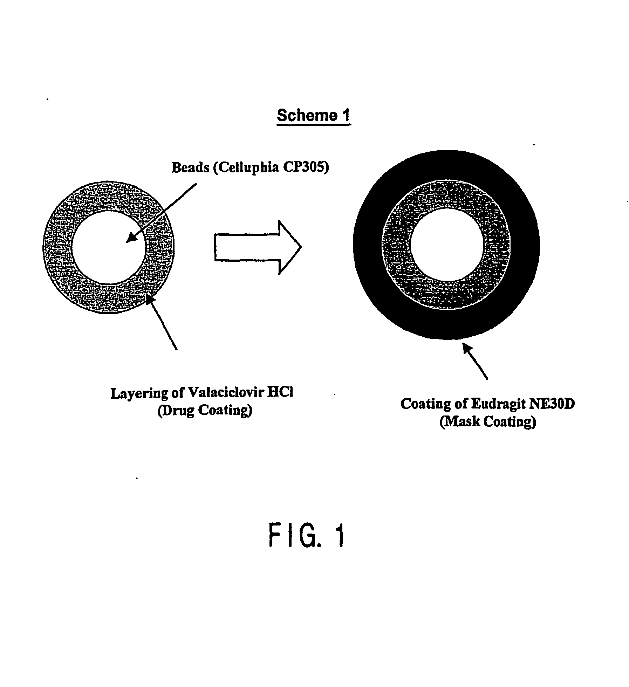

Fig. 1 is Scheme 1 and shows the preparation of carrier beads of Celluphia CP305 coated with a drug coat layer comprising valaciclovir HCI to which a mask coat layer comprising Eudragit NE30D is applied.

### Example 2

| | | | |
|---|---|---|---|
| Carrier Beads | Sucrose | Nonpareils #32-42 | 20-40% |
| | | Nonpareils#20-40 | |
| | | Nonpareils#20-24 | |
| | Lactose | Lactose#50 | |
| | | Lactose#30-50 | |
| | Microcrystalline cellulose | Celluphia CP203 | |
| Binders in the drug coat layer the drug | Cellulosic | HPC-L | 1-30 % |
| | Starch | Com Starch | |
| | Povidone (PVP) | PVP K90 | |
| Polymers in the mask coat layer | Eudragit | L100 | 1-25% |
| | | L30D55 | |
| | | RS30D | |
| | Cellulosic | Ethyl Cellulose | |
| Binder/dissolution enhancers in the mask coat layer | Cellulosic | HPMC | 0.5-10% |
| | Others | PEG 6000 | |
| | | Polysorbate 80 | |
| | | Triacetin | |
| | | Titanium | |
| | | Dioxide | |
| | | SLS | |
| | | TEC | |

### 1) Drug Coating

As alternative carrier beads, Nonpareils (#32-42, #20-40, #20-24), Lactose (#50, #30-50) or Celluphia CP203 may be used. The amount of these ingredients are 20 to 40% by weight of the total formulation. HPC-L, Corn Starch or PVP-K90 can be used as a binder and the amount is 1 to 30% by weight of the total formulation.

### 2) Mask Coating

As alternative mask coat polymers, Eudragit (L100, L30D55, RS30D) or Ethyl Cellulose may be used. The amount of these ingredients are 1 to 30%, e.g. 1 to 25%, by weight of the total formulation. Other binder/dissolution enhancers (HPMC, Polyethyleneglycol 6000 (PEG 6000), Polysorbate 80, Triacetin, Titanium Dioxide, Sodium Lauryl Sulphate (SLS) or Triethyl citrate (TEC)) can be used and the amount is 0.1 to 20%, e.g. 0.5 to 10%, by weight of the total formulation.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

The application of which this description and claims forms part may be used as a basis for priority in respect of any subsequent application. The claims of such subsequent application may be directed to any novel feature or combination of features described herein. This may take the form of product, composition, process or use claims and may include, by way of example and without limitation, one or more of the following claims.

## Claims

1. A pharmaceutical formulation comprising carrier beads coated with one or more layers of pharmaceutically acceptable substances,
wherein, at least one layer is a drug coat layer comprising valaciclovir, or a pharmaceutically acceptable derivative thereof, as the active ingredient, wherein "pharmaceutically acceptable derivative" means any pharmaceutically acceptable salt of valaciclovir which, upon administration to the recipient, is capable of providing (directly or indirectly) valaciclovir or an antivirally active metabolite or residue thereof; and
wherein the pharmaceutical formulation further comprises a mask coat layer.

2. A pharmaceutical formulation as claimed in claim 1, wherein the coated carrier beads are granules in which:
(i) no granules have a particle size greater than 1700µm (micrometres),
(ii) no more than 5% by weight of the granules have a particle size greater than 1400µm (micrometres), and
(iii) no more than 15% by weight of the granules have a particle size smaller than 355µm (micrometres).

3. A pharmaceutical formulation as claimed in claim 2, wherein no more than 5% by weight of the granules have a particle size smaller than 355µm (micrometres).

4. A pharmaceutical formulation as claimed in claim 2 or 3, which has the following properties:
(i) no granules have a particle size greater than 1700µm (micrometres).
(ii) at least 95% by weight of the granules have a particle size of from 355µm (micrometres) to 1400µm (micrometres), and
(iii) no more than 0.5% by weight of the granules have a particle size smaller than 355µm (micrometres).

5. A pharmaceutical formulation as claimed in claim 2, 3 or 4, wherein the range of particle sizes for the finished granules is 355 to 850µm (micrometres).

6. A pharmaceutical formulation as claimed in claim 2, 3, 4 or 5, wherein the carrier beads have an average particle size of 300 to 500µm (micrometres).

7. A pharmaceutical formulation as claimed in claim 1, 2, 3, 4, 5 or 6, wherein the mask coat layer comprises a polymer, a component which acts as a lubricant and a component which acts as a binder/dissolution enhancer.

8. A pharmaceutical formulation as claimed in claim 7, wherein the polymer includes a methacrylic acid copolymer, Hydroxy Propyl Cellulose, Hydroxy Propyl Methyl Cellulose (HPMC), Hydroxy Propyl Methyl Cellulose Phthalate (HPMCP), Ethyl Cellulose (EC), Carboxy Methyl Ethyl Cellulose (CMEC), Cellulose Acetate Phthalate (CAP), Hydroxy Ethyl Cellulose (HEC), wax, gelatin, or shellac.

9. A pharmaceutical formulation as claimed in claim 8, wherein the polymer includes a methacrylic acid copolymer.

10. A pharmaceutical formulation as claimed in claim 9, wherein the methacrylic acid copolymer is Eudragit NE30D, L30D55, L10055, L100, E100, S100, RS100L, RS30DL, RS100 or RS30D.

11. A pharmaceutical formulation as claimed in claim 9, wherein the methacrylic acid copolymer is Eudragit NE30D.

12. A pharmaceutical formulation as claimed in claim 7, 8, 9, 10 or 11, wherein the polymer is present in amounts of 1 to 30% by weight of the total formulation.

13. A pharmaceutical formulation as claimed in claim 12, wherein the polymer is present in amounts of 1 to 10% by weight of the total formulation.

14. A pharmaceutical formulation as claimed in any of claims 7 to 13, wherein the lubricant is present in amounts of 1 to 10% by weight of the total formulation.

15. A pharmaceutical formulation as claimed in any of claims 7 to 14, wherein the binder/dissolution enhancer includes Hydroxy Propyl Cellulose (HPC-L), polyvinylpyrrolidone (PVP), starch, Hydroxy Propyl Methyl Cellulose (HPMC). Polyethylene glycol 6000 (PEG 6000), Polysorbate 80, Triacetin, Titanium Dioxide or Sodium Lauryl Sulphate.

16. A pharmaceutical formulation as claimed in claim 15, wherein the binder/dissolution enhancer is Hydroxy Propyl Cellulose (HPC-L).

17. A pharmaceutical formulation as claimed in any of claims 7 to 16, wherein the binder/dissolution enhancer is present in amounts of 0.1 to 20% by weight of the total formulation.

18. A pharmaceutical formulation as claimed in claim 17, wherein the binder/dissolution enhancer is present in amounts of 0.1 to 5% by weight of the total formulation.

19. A pharmaceutical formulation as claimed in any of claims 1 to 18, which further comprises a mask coat layer, and
in the formulation, carrier beads of Celluphia CP305 cellulose have been coated with a drug coat layer comprising valaciclovir HCl to which a mask coat layer comprising Eudragit NE30D has been applied.

20. A pharmaceutical formulation as claimed in any preceding claim, containing 1 to 90% by weight of the valaciclovir or the pharmaceutically acceptable derivative thereof.

21. A pharmaceutical formulation as claimed in claim 20, containing 25 to 75% by weight of the valaciclovir or the pharmaceutically acceptable derivative thereof.

22. A pharmaceutical formulation as claimed in any preceding claim, wherein the valaciclovir or the pharmaceutically acceptable derivative thereof is valaciclovir or a pharmaceutically acceptable acid addition salt of valaciclovir derived from an appropriate acid.

23. A pharmaceutical formulation to any preceding claim, wherein the carrier beads comprise cellulose, lactose, starch or non-pareils (wherein non-pareils are carrier beads made of sucrose).

24. A pharmaceutical formulation as claimed in claim 23, wherein the carrier beads comprise cellulose.

25. A pharmaceutical formulation as claimed in claim 24, wherein the carrier beads comprise Celluphia CP305 cellulose or Celluphia CP203 cellulose.

26. A pharmaceutical formulation as claimed in claim 24, wherein the carrier beads comprise Celluphia CP305 cellulose.

27. A pharmaceutical formulation as claimed in claim 24, wherein the carrier beads comprise microcrystalline cellulose.

28. A pharmaceutical formulation as claimed in claim 23, 24, 25, 26 or 27,
wherein the carrier beads are present in amounts of 1 to 50% by weight of the total formulation.

29. A pharmaceutical formulation as claimed in claim 28, wherein the carrier beads are present in amounts of 20 to 50% by weight of the total formulation.

30. A pharmaceutical formulation as claimed in claim 28, wherein the carrier beads are present in amounts of 20 to 40% by weight of the total formulation.

31. A pharmaceutical formulation as claimed in any of claims 23 to 30, wherein the carrier beads have an average particle size of 300 to 500µm (micrometres).

32. A pharmaceutical formulation as claimed in claim 26, 28, 29, 30 or 31,
wherein carrier beads of Celluphia CP305 cellulose have been coated with a drug coat layer comprising valaciclovir HCl to which a mask coat layer comprising Eudragit NE30D has been applied.

33. A pharmaceutical formulation as claimed in any preceding claim, wherein the drug coat layer further comprises a component which acts as a binder.

34. A pharmaceutical formulation as claimed in claim 33, wherein the binder includes Hydroxy Propyl Cellulose (HPC-L), Hydroxy Propyl Methyl Cellulose (HPMC), starch, or polyvinylpyrrolidone (PVP).

35. A pharmaceutical formulation as claimed in claim 34, wherein the binder includes polyvinylpyrrolidone (PVP).

36. A pharmaceutical formulation as claimed in claim 35, wherein the binder includes polyvinylpyrrolidone grade K30 (PVP K30).

37. A pharmaceutical formulation as claimed in claim 33, 34, 35 or 36, wherein the binder is present in amounts of 1 to 30% by weight of the total formulation.

38. A pharmaceutical formulation as claimed in claim 37, wherein the binder is present in amounts of 1 to 10% by weight of the total formulation.

39. A pharmaceutical formulation as claimed in any one of claims 1 to 38, further comprising a terminal blend.

40. A pharmaceutical formulation as claimed in claim 39, wherein the terminal blend comprises one or more lubricants selected from talc, aerosil, kaolin, magnesium stearate and stearic acid.

41. A pharmaceutical formulation as claimed in claim 39 or 40, wherein the terminal blend comprises 0.1 to 10% by weight of the total formulation.

42. A pharmaceutical formulation as claimed in any one of claims 1 to 41, which comprises:
(a) carrier beads, comprising from 1 to 50% by weight of the total formulation:
(b) a drug coat layer comprising from 1 to 90% by weight of the total formulation comprising valaciclovir, or a pharmaceutically acceptable derivative thereof, and from 1 to 30% by weight of the total formulation of a component which acts as a binder:
(c) a mask coat layer comprising from 1 to 30% by weight of the total formulation of a polymer, from 1 to 30% by weight of the total formulation of a component which acts as a lubricant and from 0.1 to 20% by weight of the total formulation of a component which acts as a binder/dissolution enhancer, and
(d) a terminal blend comprising from 0.1 to 20% by weight of the total formulation of a component which acts as a lubricant.

43. A pharmaceutical formulation as claimed in claim 42, which comprises:
(a) carrier beads, comprising from 20 to 40% by weight of the total formulation;
(b) a drug coat layer comprising from 25 to 75% by weight of the total formulation comprising valaciclovir, or a pharmaceutically acceptable derivative thereof, and from 1 to 10% by weight of the total formulation of a component which acts as a binder:
(c) a mask coat layer comprising from 1 to 10% by weight of the total formulation of a polymer, from 1 to 10% by weight of the total formulation of a component which acts as a lubricant, and from 0.1 to 5% by weight of the total formulation of a component which acts as a binder/dissolution enhancer; and
(d) a terminal blend comprising from 0.1 to 10% by weight of the total formulation of a component which acts as a lubricant.

44. A pharmaceutical formulation as claimed in claim 43, which comprises:
(a) cellulose carrier beads, comprising about 30.6% by weight of the total formulation;
(b) a drug coat layer comprising about 55.6% by weight of the total formulation of valaciclovir hydrochloride, and about 4.2% by weight of the total formulation of polyvinylpyrrolidone as a binder;
(c) a mask coat layer comprising about 4.11 % by weight of the total formulation of methacrylic acid copolymer, about 4.11% by weight of the total formulation of talc as a lubricant, and about 0.89% by weight of the total formulation of hydroxypropylcellulose as a binder/dissolution enhancer; and
(d) a terminal blend comprising about 0.25% by weight of the total formulation of talc and 0.25% by weight of the total formulation of aerosil which act as a lubricant.

45. A pharmaceutical formulation as claimed in any one of claims 1 to 44 together with one or more pharmaceutically acceptable carriers or excipients.

46. Use of a pharmaceutical formulation as claimed in any one of claims 1 to 45 in the manufacture of a medicament for the treatment of viral infections.

47. The use as claimed in claim 46 wherein the viral infection is caused by a member of the herpes family of viruses.

48. A process for preparing a pharmaceutical formulation as claimed in any one of claims 1 to 45 comprising coating a carrier bead with a drug coat layer comprising valaciclovir, or a pharmaceutically acceptable derivative thereof, and further coating with a mask coat layer;
wherein "pharmaceutically acceptable derivative" means any pharmaceutically acceptable salt of valaciclovir which, upon administration to the recipient, is capable of providing (directly or indirectly) valaciclovir or an antivirally active metabolite or residue thereof.

49. A process as claimed in claim 48, wherein the one or more layers of the pharmaceutically acceptable substances are applied to carrier beads using either a non-aqueous system with ethanol or isopropyl alcohol (IPA) as a solvent, or alternatively using an aqueous system.

50. A process for preparing a pharmaceutical formulation as claimed in claim 48
wherein the drug coat layer is applied to the carrier bead using an aqueous solvent.

51. A process as claimed in claim 50, wherein water is used as the solvent to form the layer of the valaciclovir or the pharmaceutically acceptable derivative thereof.

52. A process as claimed in any of claims 48 to 51 wherein the drug coat layer is prepared by:
(a) first heating a or the solvent and dissolving the valaciclovir, or the pharmaceutical acceptable derivative thereof, and a binder in the solvent,
(b) then cooling the solution to room temperature and filtering, and
(c) spraying the obtained aqueous suspension (the valaciclovir, or the derivative thereof, and the binder) onto the carrier beads, and
(d) drying the coated carrier beads in a rotated fluid bed granulator.

53. A process as claimed in any of claims 48 to 52, wherein a or the mask coat layer is prepared by:
(a) mixing a polymer, a lubricant, a binder/dissolution enhancer and a solvent, to form a suspension, and filtering the suspension;
(b) spraying the mask coat suspension onto the drug-coated beads; and
(c) drying the coated carrier beads in a rotated fluid bed granulator.

54. A process as claimed in claim 53, wherein wherein the mask coat layer is prepared by (a) mixing the polymer, the lubricant, the binder/dissolution enhancer and purified water as the solvent, to form a suspension, and filtering the suspension.

55. A process as claimed in any of claims 48 to 54, wherein the mask coat layer is as defined in any of claims 7 to 18.

56. A process as claimed in any of claims 48 to 55, wherein the carrier bead is as defined in any of claims 23 to 31.

57. A process as claimed in any of claims 48 to 56, wherein the particle size is as defined in any of claims 2 to 6.

58. A process as claimed in any of claims 48 to 57, comprising further coating with a terminal blend.

## Patentansprüche

1. Pharmazeutische Formulierung, umfassend Trägerkügelchen, die mit einer oder mehreren Lagen pharmazeutisch verträglicher Stoffe beschichtet sind,
wobei mindestens eine Schicht eine Arzneistoffüberzugsschicht ist, umfassend Valaciclovir oder ein pharmazeutisch verträgliches Derivat hiervon als Wirkstoff, wobei "pharmazeutisch verträgliches Derivat" jegliches pharmazeutisch verträgliche Salz von Valaciclovir bedeutet, das, nach Verabreichung an den Empfänger, in der Lage ist (direkt oder indirekt), Valaciclovir bereitzustellen oder einen antiviral aktiven Metaboliten oder Rest hiervon; und
wobei die pharmazeutische Formulierung ferner eine maskierende Überzugsschicht umfasst.

2. Pharmazeutische Formulierung wie in Anspruch 1 beansprucht, wobei die beschichteten Trägerkügelchen Granula sind, wobei:
(i) keine der Granula eine Teilchengröße haben, die größer als 1700 µm (Mikrometer) ist,
(ii) nicht mehr als 5 Gewichtsprozent der Granula eine Teilchengröße haben, die größer als 1400 µm (Mikrometer) ist, und
(iii) nicht mehr als 15 Gewichtsprozent der Granula eine Teilchengröße haben, die kleiner als 355 µm (Mikrometer) ist.

3. Pharmazeutische Formulierung wie in Anspruch 2 beansprucht, wobei nicht mehr als 5 Gewichtsprozent der Granula eine Teilchengröße haben, die kleiner als 355 µm (Mikrometer) ist.

4. Pharmazeutische Formulierung wie in Anspruch 2 oder 3 beansprucht, die die folgenden Eigenschaften hat:
(i) keine der Granula haben eine Teilchengröße, die größer als 1700 µm (Mikrometer) ist,
(ii) mindestens 95 Gewichtsprozent der Granula haben eine Teilchengröße von 355 µm (Mikrometer) bis 1400 µm (Mikrometer), und
(iii) nicht mehr als 0,5 Gewichtsprozent der Granula haben eine Teilchengröße, die kleiner als 355 µm (Mikrometer) ist.

5. Pharmazeutische Formulierung wie in Anspruch 2, 3 oder 4 beansprucht, wobei der Bereich der Teilchengrößen für die fertigen Granula von 355 bis 850 µm (Mikrometer) ist.

6. Pharmazeutische Formulierung wie in Anspruch 2, 3, 4 oder 5 beansprucht, wobei die Trägerkügelchen eine durchschnittliche Teilchengröße von 300 bis 500 µm (Mikrometer) haben.

7. Pharmazeutische Formulierung wie in Anspruch 1, 2, 3, 4, 5 oder 6 beansprucht, wobei die maskierende Überzugsschicht ein Polymer, eine Komponente, die als Gleitmittel wirkt, und eine Komponente, die als Bindemittel/Auflösungsverstärker wirkt, umfasst.

8. Pharmazeutische Formulierung wie in Anspruch 7 beansprucht, wobei das Polymer einschließt: ein Methacrylsäurecopolymer, Hydroxypropylcellulose, Hydroxypropylmethylcellulose (HPMC), Hydroxypropylmethylcellulosephthalat (HPMCP), Ethylcellulose (EC), Carboxymethylethylcellulose (CMEC), Celluloseacetatephthalat (CAP), Hydroxyethylcellulose (HEC), Wachs, Gelatine, oder Schellack.

9. Pharmazeutische Formulierung wie in Anspruch 8 beansprucht, wobei das Polymer ein Methacrylsäurecopolymer einschließt.

10. Pharmazeutische Formulierung wie in Anspruch 9 beansprucht, wobei das Methacrylsäurecopolymer Eudragit NE30D, L30D55, L10055, L100, E100, S100, RS100L, RS30DL, RS100 oder RS30D ist.

11. Pharmazeutische Formulierung wie in Anspruch 9 beansprucht, wobei das Methacrylsäurecopolymer Eudragit NE30D ist.

12. Pharmazeutische Formulierung wie in den Ansprüchen 7, 8, 9, 10 oder 11 beansprucht, wobei das Polymer in Mengen von 1 bis 30 Gewichtsprozent der Gesamtformulierung vorliegt.

13. Pharmazeutische Formulierung wie in Anspruch 12 beansprucht, wobei das Polymer in Mengen von 1 bis 10 Gewichtsprozent der Gesamtformulierung vorliegt.

14. Pharmazeutische Formulierung wie in einem der Ansprüche 7 bis 13 beansprucht, wobei das Gleitmittel in Mengen von 1 bis 10 Gewichtsprozent der Gesamtformulierung vorliegt.

15. Pharmazeutische Formulierung wie in einem der Ansprüche 7 bis 14 beansprucht, wobei das Bindemittel/der Auflösungsverstärker einschließt: Hydroxypropylcellulose (HPC-L), Polyvinylpyrrolidon (PVP), Stärke, Hydroxypropylmethylcellulose (HPMC), Polyethylenglycol 6000 (PEG 6000), Polysorbat 80, Triacetin, Titandioxid oder Natriumlaurylsulfat.

16. Pharmazeutische Formulierung wie in Anspruch 15 beansprucht, wobei das Bindemittel/der Auflösungsverstärker Hydroxypropylcellulose (HPC-L) ist.

17. Pharmazeutische Formulierung wie in einem der Ansprüche 7 bis 16 beansprucht, wobei das Bindemittel/der Auflösungsverstärker in Mengen von 0,1 bis 20 Gewichtsprozent der Gesamtformulierung vorliegt.

18. Pharmazeutische Formulierung wie in Anspruch 17 beansprucht, wobei das Bindemittel/der Auflösungsverstärker in Mengen von 0,1 bis 5 Gewichtsprozent der Gesamtformulierung vorliegt.

19. Pharmazeutische Formulierung wie in einem der Ansprüche 1 bis 18 beansprucht, die ferner eine maskierende Überzugsschicht umfasst, und wobei in der Formulierung Trägerkügelchen aus Celluphia CP305-Cellulose mit einer Arzneistoffüberzugsschicht beschichtet wurden, die Valaciclovir-HCl umfasst, auf die eine maskierende Überzugsschicht umfassend Eudragit NE30D aufgebracht wurde.

20. Pharmazeutische Formulierung wie in einem der vorangegangenen Ansprüche beansprucht, enthaltend 1 bis 90 Gewichtsprozent von Valaciclovir oder dem pharmazeutisch verträglichen Derivat hiervon.

21. Pharmazeutische Formulierung wie in Anspruch 20 beansprucht, enthaltend 20 bis 75 Gewichtsprozent von Valaciclovir oder dem pharmazeutisch verträglichen Derivat hiervon.

22. Pharmazeutische Formulierung wie in einem der vorangegangenen Ansprüche beansprucht, wobei Valaciclovir oder das pharmazeutisch verträgliche Derivat hiervon Valaciclovir ist oder ein pharmazeutisch verträgliches Säureadditionssalz von Valaciclovir, das von einer geeigneten Säure abgeleitet ist.

23. Pharmazeutische Formulierung nach einem der vorangegangenen Ansprüche, wobei die Trägerkügelchen Cellulose, Lactose, Stärke oder Non-Pareils (wobei Non-Pareils aus Saccharose hergestellte Trägerkügelchen sind) umfassen.

24. Pharmazeutische Formulierung wie in Anspruch 23 beansprucht, wobei die Trägerkügelchen Cellulose umfassen.

25. Pharmazeutische Formulierung wie in Anspruch 24 beansprucht, wobei die Trägerkügelchen Celluphia CP305-Cellulose oder Celluphia CP203-Cellulose umfassen.

26. Pharmazeutische Formulierung wie in Anspruch 24 beansprucht, wobei die Trägerkügelchen Celluphia CP305-Cellulose umfassen.

27. Pharmazeutische Formulierung wie in Anspruch 24 beansprucht, wobei die Trägerkügelchen mikrokristalline Cellulose umfassen.

28. Pharmazeutische Formulierung wie in einem der Ansprüche 23, 24, 25, 26 oder 27 beansprucht, wobei die Trägerkügelchen in Mengen von 1 bis 50 Gewichtsprozent der Gesamtformulierung vorliegen.

29. Pharmazeutische Formulierung wie in Anspruch 28 beansprucht, wobei die Trägerkügelchen in Mengen von 20 bis 50 Gewichtsprozent der Gesamtformulierung vorliegen.

30. Pharmazeutische Formulierung wie in Anspruch 28 beansprucht, wobei die Trägerkügelchen in Mengen von 20 bis 40 Gewichtsprozent der Gesamtformulierung vorliegen.

31. Pharmazeutische Formulierung wie in einem der Ansprüche 23 bis 30 beansprucht, wobei die Trägerkügelchen eine durchschnittliche Teilchengröße von 300 bis 500 µm (Mikrometer) haben.

32. Pharmazeutische Formulierung wie in Anspruch 26, 28, 29, 30 oder 31 beansprucht, wobei Trägerkügelchen aus Celluphia CP305-Cellulose mit einer Arzneistoffüberzugsschicht umfassend Valaciclovir-HCl beschichtet wurden, auf die eine maskierende Überzugsschicht umfassend Eudragit NE30D aufgebracht wurde.

33. Pharmazeutische Formulierung wie in einem der vorangegangen Ansprüche beansprucht, wobei die Arzneistoffüberzugsschicht ferner eine Komponente umfasst, die als Bindemittel wirkt.

34. Pharmazeutische Formulierung wie in Anspruch 33 beansprucht, wobei das Bindemittel einschließt: Hydroxypropylcellulose (HPC-L), Hydroxypropylmethylcellulose (HPMC) Stärke oder Polyvinylpyrrolidon (PVP).

35. Pharmazeutische Formulierung wie in Anspruch 34 beansprucht, wobei das Bindemittel Polyvinylpyrrolidon (PVP) einschließt.

36. Pharmazeutische Formulierung wie in Anspruch 35 beansprucht, wobei das Bindemittel Polyvinylpyrrolidon-Grad K30 (PVP K30) einschließt.

37. Pharmazeutische Formulierung wie in Anspruch 33, 34, 35 oder 36 beansprucht, wobei das Bindemittel in Mengen von 1 bis 30 Gewichtsprozent der Gesamtformulierung vorliegt.

38. Pharmazeutische Formulierung nach Anspruch 37, wobei das Bindemittel in Mengen von 1 bis 10 Gewichtsprozent der Gesamtformulierung vorliegt.

39. Pharmazeutische Formulierung wie in einem der Ansprüche 1 bis 38 beansprucht, ferner eine abschließende Beschichtung (terminal blend) umfassend.

40. Pharmazeutische Formulierung wie in Anspruch 39 beansprucht, wobei die abschließende Beschichtung ein oder mehrere Gleitmittel umfasst, ausgewählt aus Talk, Aerosil, Kaolin, Magnesiumstearat und Stearinsäure.

41. Pharmazeutische Formulierung wie in Anspruch 39 oder 40 beansprucht, wobei die abschließende Beschichtung 0,1 bis 10 Gewichtsprozent der Gesamtformulierung umfasst.

42. Pharmazeutische Formulierung wie in einem der Ansprüche 1 bis 41 beansprucht, die umfasst:
(a) Trägerkügelchen, umfassend 1 bis 50 Gewichtsprozent der Gesamtformulierung;
(b) eine Arzneistoffüberzugsschicht, umfassend 1 bis 90 Gewichtsprozent der Gesamtformulierung, umfassend Valaciclovir oder ein pharmazeutisch verträgliches Derivat hiervon, und eine Komponente, die als Bindemittel wirkt, in einer Menge von 1 bis 30 Gewichtsprozent der Gesamtformulierung;
(c) eine maskierende Überzugsschicht, umfassend ein Polymer in einer Menge von 1 bis 30 Gewichtsprozent der Gesamtformulierung, eine Komponente, die als Gleitmittel wirkt, in einer Menge von 1 bis 30 Gewichtsprozent der Gesamtformulierung und eine Komponente, die als Bindemittel/Auflösungsverstärker wirkt, in einer Menge von 0,1 bis 20 Gewichtsprozent der Gesamtformulierung; und
(d) eine abschließende Beschichtung (terminal blend), umfassend eine Komponente, die als Gleitmittel wirkt, in einer Menge von 0,1 bis 20 Gewichtsprozent der Gesamtformulierung.

43. Pharmazeutische Formulierung wie in Anspruch 42 beansprucht, die umfasst:
(a) Trägerkügelchen, umfassend 20 bis 40 Gewichtsprozent der Gesamtformulierung;
(b) eine Arzneistoffüberzugsschicht, umfassend 25 bis 75 Gewichtsprozent der Gesamtformulierung, umfassend Valaciclovir oder ein pharmazeutisch verträgliches Derivat hiervon, und eine Komponente, die als Bindemittel wirkt, in einer Menge von 1 bis 10 Gewichtsprozent der Gesamtformulierung;
(c) eine maskierende Überzugsschicht, umfassend ein Polymer in einer Menge von 1 bis 10 Gewichtsprozent der Gesamtformulierung, eine Komponente, die als Gleitmittel wirkt, in einer Menge von 1 bis 10 Gewichtsprozent der Gesamtformulierung und eine Komponente, die als Bindemittel/Auflösungsverstärker wirkt, in einer Menge von 0,1 bis 5 Gewichtsprozent der Gesamtformulierung; und
(d) eine abschließende Beschichtung (terminal blend), umfassend eine Komponente, die als Gleitmittel wirkt, in einer Menge von 0,1 bis 10 Gewichtsprozent der Gesamtformulierung.

44. Pharmazeutische Formulierung wie in Anspruch 43 beansprucht, die umfasst:
(a) Celluloseträgerkügelchen, umfassend etwa 30,6 Gewichtsprozent der Gesamtformulierung;
(b) eine Arzneistoffüberzugsschicht, umfassend Valacielovirhydrochlorid in einer Menge von etwa 55,6 Gewichtsprozent der Gesamtformulierung, und Polyvinylpyrrolidon als Bindemittel in einer Menge von etwa 4,2 Gewichtsprozent der Gesamtformulierung;
(c) eine maskierende Überzugsschicht, umfassend Methacrylsäurecopolymer in einer Menge von etwa 4,11 Gewichtsprozent der Gesamtformulierung, Talk als Gleitmittel in einer Menge von etwa 4,11 Gewichtsprozent der Gesamtformulierung und Hydroxypropylcellulose als Bindemittel/Auflösungsverstärker in einer Menge von etwa 0,89 Gewichtsprozent der Gesamtformulierung; und
(d) eine abschließende Beschichtung (terminal blend), umfassend Talk in einer Menge von etwa 0,25 Gewichtsprozent der Gesamtformulierung und Aerosil, dass als Gleitmittel wirkt, in einer Menge von 0,25 Gewichtsprozent der Gesamtformulierung.

45. Pharmazeutische Formulierung wie in einem der Ansprüche 1 bis 44 beansprucht, zusammen mit einem oder mehreren pharmazeutisch verträglichen Trägern oder Exzipienten.

46. Verwendung einer pharmazeutischen Formulierung wie in einem der Ansprüche 1 bis 45 beansprucht, für die Herstellung eines Medikaments zur Behandlung viraler Infektionen.

47. Verwendung wie in Anspruch 46 beansprucht, wobei die virale Infektion von einem Mitglied der Familie der Herpesviren verursacht ist.

48. Verfahren zur Herstellung einer pharmazeutischen Formulierung wie in einem der Ansprüche 1 bis 45 beansprucht, umfassend das Beschichten eines Trägerkügelchens mit einer Arzneistoffüberzugsschicht, umfassend Valaciclovir oder ein pharmazeutisch verträgliches Derivat hiervon, und ferner das Beschichten mit einer maskierenden Überzugsschicht;
wobei "pharmazeutisch verträgliches Derivat" jegliches pharmazeutisch verträgliche Salz von Valaciclovir bedeutet, das, nach Verabreichung an den Empfänger, in der Lage ist (direkt oder indirekt), Valaciclovir oder einen antiviral aktiven Metaboliten oder Rest hiervon bereitzustellen.

49. Verfahren wie in Anspruch 48 beansprucht, wobei die eine oder die mehreren Schichten der pharmazeutisch verträglichen Stoffe auf die Trägerkügelchen aufgebracht werden unter Verwendung entweder eines nicht-wässrigen Systems mit Ethanol oder Isopropylalkohol (IPA) als Lösungsmittel, oder alternativ unter Verwendung eines wässrigen Systems.

50. Verfahren zur Herstellung einer pharmazeutischen Formulierung wie in Anspruch 48 beansprucht, wobei die Arzneistoffüberzugsschicht auf das Trägerkügelchen unter Verwendung eines wässrigen Lösungsmittels aufgebracht wird.

51. Verfahren wie in Anspruch 50 beansprucht, wobei Wasser als Lösungsmittel verwendet wird, um die Schicht von Valaciclovir oder dem pharmazeutisch verträglichen Derivat hiervon zu erzeugen.

52. Verfahren wie in einem der Ansprüche 48 bis 51 beansprucht, wobei die Arzneistoffüberzugsschicht hergestellt wird durch:
(a) zuerst Erhitzen eines oder des Lösungsmittels und Auflösen von Valaciclovir oder des pharmazeutisch verträglichen Derivats hiervon und eines Bindemittels in dem Lösungsmittel,
(b) Abkühlen der Lösung auf Raumtemperatur und Filtrieren, und
(c) Sprühen der erhaltenen wässrigen Suspension (das Valaciclovir oder das Derivat hiervon und das Bindemittel) auf die Trägerkügelchen, und
(d) Trocknen der beschichteten Trägerkügelchen in einem rotierenden Wirbelschichtgranulator.

53. Verfahren wie in einem der Ansprüche 48 bis 52 beansprucht, wobei eine oder die maskierende Überzugsschicht hergestellt wird durch:
(a) Mischen eines Polymers, eines Gleitmittels, eines Bindemittels/Auflösungsverstärkers und eines Lösungsmittels, um eine Suspension zu erzeugen, und Filtrieren der Suspension;
(b) Sprühen der maskierenden Überzugssuspension auf die arzneistoffbeschichteten Kügelchen, und
(c) Trocknen der beschichteten Trägerkügelchen in einem rotierenden Wirbelschichtgranulator.

54. Verfahren wie in Anspruch 53 beansprucht, wobei die maskierende Überzugsschicht hergestellt wird durch (a) Mischen des Polymers, des Gleitmittels, des Bindemittels/Auflösungsverstärkers und gereinigten Wassers als Lösungsmittel, um eine Suspension zu erzeugen, und Filtrieren der Suspension.

55. Verfahren wie in einem der Ansprüche 48 bis 54 beansprucht, wobei die maskierende Überzugsschicht so ist, wie in einem der Ansprüche 7 bis 18 definiert.

56. Verfahren wie in einem der Ansprüche 48 bis 55 beansprucht, wobei das Trägerkügelchen so ist, wie in einem der Ansprüche 23 bis 31 definiert.

57. Verfahren wie in einem der Ansprüche 48 bis 56 beansprucht, wobei die Teilchengröße so ist, wie in einem der Ansprüche 2 bis 6 definiert.

58. Verfahren wie in einem der Ansprüche 48 bis 57 beansprucht, ferner umfassend das Beschichten mit einer abschließenden Beschichtung (terminal blend).

## Revendications

1. Formulation pharmaceutique comprenant des billes de support enrobées d'une ou plusieurs couches de substances pharmaceutiquement acceptables,
dans laquelle au moins une couche est une couche d'enrobage de médicament comprenant du valaciclovir, ou un de ses dérivés pharmaceutiquement acceptables, comme ingrédient actif, l'expression "dérivé pharmaceutiquement acceptable" désignant n'importe quel sel pharmaceutiquement acceptable de valaciclovir qui, par administration au receveur, est capable de fournir (directement ou indirectement) du valaciclovir ou un de ses métabolites ou résidus à activité antivirale ; et
dans laquelle la formulation pharmaceutique comprend en outre une couche d'enrobage de masquage.

2. Formulation pharmaceutique suivant la revendication 1, dans laquelle les billes de support enrobées sont des granules, dans lesquelles :
(i) aucun granule n'a un diamètre de particules supérieur à 1700 µm (micromètres),
(ii) une proportion non supérieure à 5 % en poids des granules a un diamètre de particules supérieur à 1400 µm (micromètres), et
(iii) une proportion non supérieure à 15 % en poids des granules a un diamètre de particules inférieur à 355 µm (micromètres).

3. Formulation pharmaceutique suivant la revendication 2, dans laquelle une proportion non supérieure à 5 % en poids des granules a un diamètre de particules inférieur à 355 µm (micromètres).

4. Formulation pharmaceutique suivant la revendication 2 ou 3, qui a les propriétés suivantes :
(i) aucun granule n'a un diamètre de particules supérieur à 1700 µm (micromètres),
(ii) une proportion d'au moins 95 % en poids des granules a un diamètre de particules de 355 µm (micromètres) à 1400 µm (micromètres), et
(iii) une proportion non supérieure à 0,5 % en poids des granules a un diamètre de particules inférieur à 355 µm (micromètres).

5. Formulation pharmaceutique suivant la revendication 2, 3 ou 4, dans laquelle l'intervalle des diamètres de particules des granules finis va de 355 à 850 µm (micromètres).

6. Formulation pharmaceutique suivant la revendication 2, 3, 4 ou 5, dans laquelle les billes de support ont un diamètre moyen de particules de 300 à 500 µm (micromètres).

7. Formulation pharmaceutique suivant la revendication 1, 2, 3, 4, 5 ou 6, dans laquelle la couche d'enrobage de masquage comprend un polymère, un constituant qui joue le rôle de lubrifiant et un constituant qui joue le rôle de liant/activateur de dissolution.

8. Formulation pharmaceutique suivant la revendication 7, dans laquelle le polymère comprend un copolymère d'acide méthacrylique, l'hydroxypropylcellulose, l'hydroxypropylméthylcellulose (HPMC), le phtalate d'hydroxypropylméthylcellulose (HPMCP), l'éthylcellulose (EC), la carboxyméthyléthylcellulose (CMEC), l' acétophtalate de cellulose (CAP), l'hydroxyéthylcellulose (HEC), une cire, la gélatine ou la gomme laque.

9. Formulation pharmaceutique suivant la revendication 8, dans laquelle le polymère comprend un copolymère d'acide méthacrylique.

10. Formulation pharmaceutique suivant la revendication 9, dans laquelle le copolymère d'acide méthacrylique est l'Eudragit NE30D, L30D55, L10055, L100, E100, S100, RS100L, RS30DL, RS100 ou RS30D.

11. Formulation pharmaceutique suivant la revendication 9, dans laquelle le copolymère d'acide méthacrylique est l'Eudragit NE30D.

12. Formulation pharmaceutique suivant la revendication 7, 8, 9, 10 ou 11, dans laquelle le polymère est présent en des quantités de 1 à 30 % en poids de la formulation totale.

13. Formulation pharmaceutique suivant la revendication 12, dans laquelle le polymère est présent en des quantités de 1 à 10 % en poids de la formulation totale.

14. Formulation pharmaceutique suivant l'une quelconque des revendications 7 à 13, dans laquelle le lubrifiant est présent en des quantités de 1 à 10 % en poids de la formulation totale.

15. Formulation pharmaceutique suivant l'une quelconque des revendications 7 à 14, dans laquelle le liant/activateur de dissolution comprend l'hydroxypropylcellulose (HPC-L), la polyvinylpyrrolidone (PVP), l'amidon, l'hydroxypropylméthylcellulose (HPMC), le polyéthylèneglycol 6000 (PEG 6000), le polysorbate 80, la triacétine, le dioxyde de titane ou le laurylsulfate de sodium.

16. Formulation pharmaceutique suivant la revendication 15, dans laquelle le liant/activateur de dissolution est l'hydroxypropylcellulose (HPC-L).

17. Formulation pharmaceutique suivant l'une quelconque des revendications 7 à 16, dans laquelle le liant/activateur de dissolution est présent en des quantités de 0,1 à 20 % en poids de la formulation totale.

18. Formulation pharmaceutique suivant la revendication 17, dans laquelle le liant/activateur de dissolution est présent en des quantités de 0,1 à 5 % en poids de la formulation totale.

19. Formulation pharmaceutique suivant l'une quelconque des revendications 1 à 18, qui comprend en outre une couche d'enrobage de masquage, et
dans la formulation, des billes de support de cellulose Celluphia CP305 ont été enrobées avec une couche d'enrobage de médicament comprenant du valaciclovir.HCl auquel une couche d'enrobage de masquage comprenant de l'Eudragit NE30D a été appliquée.

20. Formulation pharmaceutique suivant l'une quelconque des revendications précédentes, contenant 1 à 90 % en poids de valaciclovir ou de son dérivé pharmaceutiquement acceptable.

21. Formulation pharmaceutique suivant la revendication 20, contenant 25 à 75 % en poids de valaciclovir ou de son dérivé pharmaceutiquement acceptable.

22. Formulation pharmaceutique suivant l'une quelconque des revendications précédentes, dans laquelle le valaciclovir ou son dérivé pharmaceutiquement acceptable est le valaciclovir ou un sel d'addition d'acide pharmaceutiquement acceptable de valaciclovir dérivé d'un acide approprié.

23. Formulation pharmaceutique suivant l'une quelconque des revendications précédentes, dans laquelle les billes de support comprennent de la cellulose, du lactose, de l'amidon ou des non-pareilles (les non-pareilles étant des billes de support constituées de saccharose).

24. Formulation pharmaceutique suivant la revendication 23, dans laquelle les billes de support comprennent de la cellulose.

25. Formulation pharmaceutique suivant la revendication 24, dans laquelle les billes de support comprennent de la cellulose Celluphia CP305 ou de la cellulose Celluphia CP203.

26. Formulation pharmaceutique suivant la revendication 24, dans laquelle les billes de support comprennent de la cellulose Celluphia CP305.

27. Formulation pharmaceutique suivant la revendication 24, dans laquelle les billes de support comprennent de la cellulose microcristalline.

28. Formulation pharmaceutique suivant la revendication 23, 24, 25, 26 ou 27, dans laquelle les billes de support sont présentes en des quantités de 1 à 50 % en poids de la formulation totale.

29. Formulation pharmaceutique suivant la revendication 28, dans laquelle les billes de support sont présentes en des quantités de 20 à 50 % en poids de la formulation totale.

30. Formulation pharmaceutique suivant la revendication 28, dans laquelle les billes de support sont présentes en des quantités de 20 à 40 % en poids de la formulation totale.

31. Formulation pharmaceutique suivant l'une quelconque des revendications 23 à 30, dans laquelle les billes de support ont un diamètre moyen de particules de 300 à 500 µm (micromètres).

32. Formulation pharmaceutique suivant la revendication 26, 28, 29, 30 ou 31, dans laquelle les billes de support de cellulose Celluphia CP305 ont été enrobées avec une couche d'enrobage de médicament comprenant du valaciclovir.HCl auquel une couche d'enrobage de masquage comprenant de l'Eudragit NE30D a été appliquée.

33. Formulation pharmaceutique suivant l'une quelconque des revendications précédentes, dans laquelle la couche d'enrobage de médicament comprend en outre un constituant qui joue le rôle de liant.

34. Formulation pharmaceutique suivant la revendication 33, dans laquelle le liant comprend l'hydroxypropylcellulose (HPC-L), l'hydroxypropylméthylcellulose (HPMC), l'amidon ou la polyvinylpyrrolidone (PVP).

35. Formulation pharmaceutique suivant la revendication 34, dans laquelle le liant comprend de la polyvinylpyrrolidone (PVP).

36. Formulation pharmaceutique suivant la revendication 35, dans laquelle le liant comprend de la polyvinylpyrrolidone de qualité K30 (PVP K30).

37. Formulation pharmaceutique suivant la revendication 33, 34, 35 ou 36, dans laquelle le liant est présent en des quantités de 1 à 30 % en poids de la formulation totale.

38. Formulation pharmaceutique suivant la revendication 37, dans laquelle le liant est présent en des quantités de 1 à 10 % en poids de la formulation totale.

39. Formulation pharmaceutique suivant l'une quelconque des revendications 1 à 38, comprenant en outre un mélange terminal.

40. Formulation pharmaceutique suivant la revendication 39, dans laquelle le mélange terminal comprend un ou plusieurs lubrifiants choisis entre le talc, l'Aerosil, le kaolin, le stéarate de magnésium et l'acide stéarique.

41. Formulation pharmaceutique suivant la revendication 39 ou 40, dans laquelle le mélange terminal représente 0,1 à 10 % en poids de la formulation totale.

42. Formulation pharmaceutique suivant l'une quelconque des revendications 1 à 41, qui comprend :
(a) des billes de support, représentant 1 à 50 % en poids de la formulation totale ;
(b) une couche d'enrobage de médicament comprenant 1 à 90 % en poids de la formulation totale comprenant valaciclovir, ou d'un de ses dérivés pharmaceutiquement acceptables, et 1 à 30 % en poids de la formulation totale d'un constituant qui joue le rôle de liant ;
(c) une couche d'enrobage de masquage comprenant 1 à 30 % en poids de la formulation totale d'un polymère, 1 à 30 % en poids de la formulation totale d'un constituant qui joue le rôle de lubrifiant et 0,1 à 20 % en poids de la formulation totale d'un constituant qui joue le rôle de liant/activateur de dissolution ; et
(d) un mélange terminal comprenant 0,1 à 20 % en poids de la formulation totale d'un constituant qui joue le rôle de lubrifiant.

43. Formulation pharmaceutique suivant la revendication 42, qui comprend :
(a) des billes de support, représentant 20 à 40 % en poids de la formulation totale ;
(b) une couche d'enrobage de médicament comprenant 25 à 75 % en poids de la formulation totale comprenant valaciclovir, ou d'un de ses dérivés pharmaceutiquement acceptables, et 1 à 10 % en poids de la formulation totale d'un constituant qui joue le rôle de liant ;
(c) une couche d'enrobage de masquage comprenant 1 à 10 % en poids de la formulation totale d'un polymère, 1 à 10 % en poids de la formulation totale d'un constituant qui joue le rôle de lubrifiant et 0,1 à 5 % en poids de la formulation totale d'un constituant qui joue le rôle de liant/activateur de dissolution ; et
(d) un mélange terminal comprenant 0,1 à 10 % en poids de la formulation totale d'un constituant qui joue le rôle de lubrifiant.

44. Formulation pharmaceutique suivant la revendication 43, qui comprend :
(a) des billes de cellulose de support, représentant environ 30,6 % en poids de la formulation totale ;
(b) une couche d'enrobage de médicament comprenant environ 55,6 % en poids de la formulation totale de chlorhydrate de valaciclovir, et environ 4,2 % en poids de la formulation totale d'un polyvinylpyrrolidone servant de liant ;
(c) une couche d'enrobage de masquage comprenant environ 4,11 % en poids de la formulation totale d'un copolymère d'acide méthacrylique, environ 4,11 % en poids de la formulation totale de talc servant de lubrifiant et environ 0,89 % en poids de la formulation totale

45. Formulation pharmaceutique suivant l'une quelconque des revendications 1 à 44, conjointement avec un ou plusieurs supports ou excipients pharmaceutiquement acceptables.

46. Utilisation d'une formulation pharmaceutique suivant l'une quelconque des revendications 1 à 45 dans la production d'un médicament destiné au traitement d'infections virales.

47. Utilisation suivant la revendication 46, dans laquelle l'infection virale est provoquée par un membre de la famille des virus de l'herpès.

48. Procédé pour la préparation d'une formulation pharmaceutique suivant l'une quelconque des revendications 1 à 45, comprenant l'enrobage d'une bille de support avec une couche d'enrobage de médicament comprenant du valaciclovir, ou un de ses dérivés pharmaceutiquement acceptables, et en outre l'enrobage avec une couche d'enrobage de masquage ; et
dans lequel l'expression "dérivé pharmaceutiquement acceptable" désigne n'importe quel sel pharmaceutiquement acceptable de valaciclovir qui, par administration au receveur, est capable de fournir (directement ou indirectement) du valaciclovir ou de ses métabolites ou résidus à activité antivirale.

49. Procédé suivant la revendication 48, dans lequel la ou les couches des substances pharmaceutiquement acceptables sont appliquées aux billes de support en utilisant un système non aqueux avec de l'éthanol ou de l'alcool isopropylique (IPA) comme solvant ou, en variante, en utilisant un système aqueux.

50. Procédé pour la préparation d'une formulation pharmaceutique suivant la revendication 48, dans lequel la couche d'enrobage de médicament est appliquée à la bille de support en utilisant un solvant aqueux.

51. Procédé suivant la revendication 50, dans lequel de l'eau est utilisée comme solvant pour former la couche de valaciclovir ou de son dérivé pharmaceutiquement acceptable.

52. Procédé suivant l'une quelconque des revendications 48 à 51, dans lequel la couche d'enrobage de médicament est préparée :
(a) en chauffant tout d'abord un ou le solvant et en dissolvant le valaciclovir, ou son dérivé pharmaceutiquement acceptable, et un liant dans le solvant,
(b) puis en refroidissant la solution à température ambiante et en la filtrant, et
(c) en pulvérisant la suspension aqueuse obtenue (le valaciclovir, ou son dérivé, et le liant) sur les billes de support, et
(d) en séchant les billes de support enrobées dans un granulateur à lit fluide rotatif.

53. Procédé suivant l'une quelconque des revendications 48 à 52, dans lequel une ou la couche d'enrobage de masquage est préparée :
(a) en mélangeant un polymère, un lubrifiant, un liant/activateur de dissolution et un solvant pour former une suspension et en filtrant la suspension ;
(b) en pulvérisant la suspension d'enrobage de masquage sur les billes enrobées de médicament ; et
(c) en séchant les billes de support enrobées dans un granulateur à lit fluide rotatif.

54. Procédé suivant la revendication 53, dans lequel la couche d'enrobage de masquage est préparée (a) en mélangeant le polymère, le lubrifiant, le liant/activateur de dissolution et de l'eau purifiée comme solvant, pour former une suspension et en filtrant la suspension.

55. Procédé suivant l'une quelconque des revendications 48 à 54, dans lequel la couche d'enrobage de masquage est telle que définie dans l'une quelconque des revendications 7 à 18.

56. Procédé suivant l'une quelconque des revendications 48 à 55, dans lequel la bille de support est telle que définie dans l'une quelconque des revendications 23 à 31.

57. Procédé suivant l'une quelconque des revendications 48 à 56, dans lequel le diamètre de particules est tel que défini dans l'une quelconque des revendications 2 à 6.

58. Procédé suivant l'une quelconque des revendications 48 à 57, comprenant en outre l'enrobage avec un mélange terminal.
